# EUROPÄISCHE PATENTSCHRIFT

(11) **EP 2 041 061 B1**
(45) Veröffentlichungstag und Bekanntmachung des Hinweises auf die Patenterteilung: **08.05.2013**
(21) Anmeldenummer: 07765650.2
(22) Anmeldetag: 27.06.2007
(51) Int. Cl.: C07C 45/85, C07C 45/34, C07C 45/62, C07C 49/607, C07C 49/413

(54) **Reinigung von Cyclododecanon durch thermisches Behandeln**
Purification of cyclododecanon via thermic treatment
Purification de la cyclododécanone par traitement thermique

(30) Priorität: 29.06.2006 EP 06116260
(43) Veröffentlichungstag der Anmeldung: 01.04.2009
(73) Patentinhaber: BASF SE, 67056 Ludwigshafen (DE)
(72) Erfinder: PINKOS, Rolf, 67098 Bad Dürkheim (DE); TEBBEN, Gerd, 68159 Mannheim (DE); MÜLLER, Christian, 68167 Mannheim (DE); RUST, Harald, 67435 Neustadt (DE)
(86) Internationale Anmeldenummer: PCT/EP2007/056393
(87) Internationale Veröffentlichungsnummer: WO 2008/000754

(56) Entgegenhaltungen:
- EP-A- 1 329 448
- EP-A2- 1 018 498
- WO-A-02/12156
- DE-A1- 1 518 566
- DE-A1- 10 344 594
- DE-A1- 10 344 595
- DATABASE WPI Week 197544 Derwent Publications Ltd., London, GB; AN 1975-73456W XP002447623 & JP 50 031145 B (ASAHI CHEM IND CO LTD) 7. Oktober 1975 (1975-10-07)

## Beschreibung

Die vorliegende Erfindung betrifft ein Verfahren zur Herstellung von Cyclododecanon, umfassend die Herstellung einer Zusammensetzung (I') mindestens enthaltend Cyclododecanon und eine Reinigung, umfassend ein thermisches Behandeln der Zusammensetzung (I') mit mindestens einem heterogenen, auf einem Träger aufgebrachten Katalysator, der mindestens ein Übergangsmetall der 8. Nebengruppe des Periodensystems der Elemente enthält, und weiteres Aufreinigen durch ein Verfahren ausgewählt aus der Gruppe bestehend aus Destillation, Extraktion und Kristallisation.

Cyclische Ketone werden für verschiedene Anwendungen in hoher Reinheit benötigt. Dabei enthalten cyclische Ketone bedingt durch das Herstellungsverfahren häufig Verunreinigungen, beispielsweise solche mit sauerstoffhaltigen Gruppen, die durch herkömmliche Reinigungsverfahren wie Destillation oder Kristallisation nur schwer zu entfernen sind. Herkömmliche Reinigungsverfahren für derartige Trennprobleme sind dadurch aufwendig und kostenintensiv.

So ist beispielsweise Cyclododecanon ein wichtiges Zwischenprodukt für die Herstellung von beispielsweise Laurinlactam, Dodecandicarbonsäure und daraus abgeleiteten Polyamiden wie beispielsweise Nylon 12 oder Nylon 6.12.

Cyclododecanon wird beispielsweise durch Luftoxidation von Cyclododecan in Anwesenheit von Borsäure zu Cyclododecylborat, Hydrolyse des Borates zu Cyclododecanol und anschließende Dehydrierung des Cyclododecanols hergestellt. Cyclododecan selbst wird weiterhin durch vollständige Hydrierung von Cyclododecatrien gewonnen. Eine Beschreibung dieses technischen Verfahrens zur Synthese von Cyclododecanon findet sich unter anderem in T. Schiffer, G. Oenbrink, "Cyclododecanol, Cyclododecanon and Laurolactam" in Ullmann's Encyclopedia of Industrial Chemistry, 6th Edition, 2000, Electronic Release, Wiley VCH.

Ein weiteres Verfahren geht von der Epoxidation von Cyclododecatrien aus, wobei aus dem Epoxid nach Hydrierung und Umlagerung Cyclododecanon erhalten wird. Derartige Verfahren sind beispielsweise in EP 1 018 498 A2 offenbart.

In DE 103 44 595 A und DE 103 44 594 A werden Verfahren zur Herstellung von Cyclododecanon beschrieben, wobei in einem Verfahrensschritt eine Oxidation mit Distickstoffmonoxid erfolgt.

Allen Verfahren gemeinsam ist, dass die Reinheit der Rohprodukte ohne zusätzliche Reinigung für einige Anwendungen nicht ausreicht. Insbesondere organische Verbindungen mit sauerstoffhaltigen Gruppen sind häufig noch in zu hohen Mengen in den erhaltenen Produkten enthalten. Deshalb ist in diesen Fällen eine sehr aufwändige Reinigung, beispielsweise durch vielstufige Destillation und/oder Kristallisation notwendig.

Der vorliegenden Erfindung lag daher die Aufgabe zu Grunde, ein Verfahren bereit zu stellen, mit dem Cyclododecanon einfach und mit geringem Aufwand in hoher Reinheit erhalten werden kann.

Erfindungsgemäß wird diese Aufgabe gelöst durch ein Verfahren zur Herstellung von Cyclododecanon, mindestens umfassend die Schritte
(a) Herstellung einer Zusammensetzung (I') mindestens enthaltend Cyclododecanon, mindestens umfassend die Stufen
   (a-1) Trimerisierung von Butadien zu Cyclododecatrien
   (a-2) Oxidation von Cyclododecatrien zu Cyclododecadienon
   (a-3) Hydrierung von Cyclododecadienon zu Cyclododecanon wobei zwischen den Stufen (a-2) und (a-3) kein Behandeln der in Stufe (a-2) erhaltenen Zusammensetzung mit mindestens einer Base erfolgt,
(b) Reinigung der Zusammensetzung (I'), mindestens umfassend die Stufen
   (i) thermisches Behandeln der Zusammensetzung (I') mit mindestens einem heterogenen, auf einem Träger aufgebrachten Katalysator, der mindestens ein Übergangsmetall der 8. Nebengruppe des Periodensystems der Elemente enthält,
   (ii) weiteres Aufreinigen durch ein Verfahren ausgewählt aus der Gruppe bestehend aus Destillation, Extraktion und Kristallisation.

Durch das erfindungsgemäße Verfahren kann Cyclododecanon mit einer Reinheit von beispielsweise > 99,5 % erhalten werden. Ferner stellt das erfindungsgemäße Verfahren eine Möglichkeit dar, die Ausbeute an Cyclododecanon zu erhöhen, da die Behandlung mit mindestens einem heterogenen, auf einem Träger aufgebrachten Katalysator, der mindestens ein Übergangsmetall der 8. Nebengruppe des Periodensystems der Elemente

enthält, im Allgemeinen sehr selektiv ist und damit bei der nachfolgenden destillativen oder kristallativen Reinigung weniger Produkt verloren geht.

Unter "Behandeln° wird im Rahmen der vorliegenden Anmeldung ein in Kontakt bringen der Zusammensetzung (I') mit mindestens einem heterogene, auf einem Träger aufgebrachten Katalysator, der mindestens ein Übergangsmetall enthält, verstanden. Die Zusammensetzung (I') wird erfindungsgemäß gemäß Stufe (i) mit einem heterogene, auf einem Träger aufgebrachten Katalysator enthaltend mindestens ein Übergangsmetall der 8. Nebengruppe des Periodensystems der Elemente thermisch behandelt.

Das erfindungsgemäße Verfahren umfasst die Stufen (i) und (ii). Gemäß Stufe (i) wird die Zusammensetzung (I') mit einem heterogene, auf einem Träger aufgebrachten Katalysator, der mindestens ein Übergangsmetall der 8. Nebengruppe des Periodensystems der Elemente enthält, thermisch behandelt. Gemäß Stufe (ii) wird die derart behandelte Zusammensetzung (I') durch Destillation, Extraktion und/oder Kristallisation weiter aufgereinigt. Dabei können die Destillation, Extraktion und/oder Kristallisation nach allen dem Fachmann bekannten üblichen Verfahren durchgeführt werden.

Geeignete Lösungsmittel für die Kristallisation gemäß Stufe (ii) sind beispielsweise Alkohole, Ether, Kohlenwasserstoffe, aromatische Kohlenwasserstoffe, Ketone, bevorzugt Toluol, Xylol, Methanol, Ethanol, Propanol, Butanol, Aceton, Diethylketon oder Methyl-tert.-butylether. Erfindungsgemäß ist es ebenso möglich, dass kein Lösungsmittel verwendet wird, sondern eine Schmelzkristallisation durchgeführt wird.

Die destillative Reinigung kann in einer oder mehreren Kolonnen erfolgen. Dabei wird bevorzugt bei Drücken zwischen 1 und 2000 mbar gearbeitet. Besonders bei cyclischen Ketonen mit mehr als 8 Kohlenstoffatomen sind Drücke zwischen 5 und 500 mbar bevorzugt, besonders bevorzugt sind 10 bis 200 mbar. Die Temperaturen (Sumpftemperatur) liegen bei 100 bis 300 °C. Bevorzugt liegt die Temperatur bei der destillativen Reinigung bei 130 bis 250 °C, besonders bevorzugt bei 150 bis 220 °C.

Gemäß einer bevorzugten Ausführungsform der Erfindung wird die destillative Reinigung bei einem Druck von 1 bis 2000 mbar, bevorzugt 5 bis 500 mbar, besonders bevorzugt 10 bis 200 mbar, und einer Sumpftemperatur von 100 bis 300 °C, bevorzugt 130 bis 250 °C, besonders bevorzugt 150 bis 220 °C durchgeführt.

Sofern bei der destillativen Reinigung nur eine Kolonne verwendet wird, wird das Wertprodukt bevorzugt über einen Seitenabzug gewonnen. Dabei ist es erfindungsgemäß möglich, das gewünschte Produkt flüssig oder gasförmig zu erhalten. Über Sumpf werden vorzugsweise Schwersieder, über Kopf vorzugsweise Leichtsieder abgetrennt. Werden zwei Kolonnen verwendet, so geht das Wertprodukt bevorzugt zusammen mit Schwersiedern bevorzugt über Sumpf in die zweite Kolonne, aus der es dann über Kopf oder wiederum als Seitenabzug gewonnen werden kann. Erfindungsgemäß können auch Trennwandkolonnen zum Einsatz kommen.

Dabei ist es erfindungsgemäß auch möglich, dass zwischen den einzelnen Schritten des Verfahrens weitere Behandlungen erfolgen. Insbesondere ist es erfindungsgemäß möglich, nach der Stufe (i) den heterogenen, auf einem Träger aufgebrachten Katalysator, der mindestens ein Übergangsmetall der 8. Nebengruppe des Periodensystems der Elemente enthält, abzutrennen, insbesondere dann, wenn der Katalysator suspendiert vorliegt.

Vor der Destillation, Extraktion bzw. Kristallisation gemäß Stufe (ii) kann es vorteilhaft sein, den Katalysator aus der behandelten Zusammensetzung (I') zu entfernen. Dies kann im Fall von heterogenen Katalysatoren beispielsweise durch Filtration geschehen. Vorteilhafterweise kann der Katalysator nach der Abtrennung erneut in die Stufe (i) eingesetzt werden. Erfindungsgemäß ist es auch möglich, dass der Katalysator nach der Abtrennung einer Zwischenbehandlung, beispielsweise einer Reinigung oder Regenerierung, unterzogen wird, bevor er erneut in die Stufe (i) eingesetzt wird.

Daher betrifft die vorliegende Erfindung gemäß einer bevorzugten Ausführungsform auch ein wie zuvor beschriebenes Verfahren, wobei der Katalysator nach der Stufe (i) abgetrennt wird und anschließend gegebenenfalls erneut in die Stufe (i) eingesetzt wird.

Die Behandlung mit einem heterogenen, auf einem Träger aufgebrachten katalysator, der mindestens ein Übergangsmetall der 8. Nebengruppe des Periodensystems der Elemente enthält, erfolgt vorzugsweise bei Temperaturen von 30 bis 350°C, beispielsweise 60 bis 350°C, insbesondere von 100 bis 270°C, besonders bevorzugt von 130 bis 260°C.

Daher betriffft die vorliegende Erfindung gemäß einer weiteren Ausführungsform auch ein wie zuvor beschriebenes Verfahren, wobei die Behandlung gemäß Stufe (i) bei einer Temperatur von 60 bis 350°C durchgeführt wird.

Es wurde überraschend gefunden, dass bei der Behandlung von Zusammensetzungen enthaltend mindestens Cyclododecanon mit einem heterogenen, auf einem Träger aufgebrachten Katalysator, der mindestens ein Übergangsmetall der 8. Nebengruppe des Periodensystems der Elemente enthält, bei einer anschließenden weiteren Aufreinigung, beispielsweise durch Destillation, Extraktion und/oder durch Kristallisation Cyclododecanon in hohen Ausbeuten in Reinheiten von über 99,5 % erhalten werden können. Dabei wird das Cyclododecanon selbst nicht oder nur sehr unwesentlich angegriffen. Erfindungsgemäß handelt es sich bei den abgetrennten Verbindungen insbesondere um Alkohole, Aldehyde und Epoxide.

Bezogen auf das in der Zusammensetzung enthaltene Cyclododecanon gehen erfindungsgemäß weniger als 10% des Cyclododecanons verloren, bevorzugt weniger als 5%, insbesondere weniger als 3%.

Dabei kann das Behandeln gemäß Stufe (i) sowohl in der Gasphase als auch in der Flüssigphase erfolgen. Der Druck ist dabei in weiten Bereichen einstellbar. Er kann beispielsweise zwischen 0,001 und 300 bar liegen, bevorzugt zwischen 0,01 und 200 bar, besonders bevorzugt zwischen 0,1 und 100 bar. Bevorzugt ist erfindungsgemäß ein Druck, bei dem gegebenenfalls entstehende Leichtsieder aus dem System destillativ entfernt werden können, d.h. bei einem Druck von beispielsweise 0,25 bis 70 bar, bevorzugt 0,35 bis 50 bar, besonders bevorzugt 0,5 bis 30 bar.

Die Behandlung gemäß Stufe (i) kann diskontinuierlich oder kontinuierlich erfolgen, wobei eine kontinuierliche Behandlung bevorzugt ist. Die Verweilzeiten liegen dabei beispielsweise zwischen 0,1 und 50 Stunden, bevorzugt zwischen 0,2 und 24 Stunden, beispielsweise zwischen 0,5 und 15 Stunden, insbesondere zwischen 1 Stunde und 19 Stunden, besonders bevorzugt zwischen 1,5 und 10 Stunden.

Daher betrifft die vorliegende Erfindung gemäß einer weiteren Ausführungsform auch ein wie zuvor beschriebenes Verfahren, wobei die Behandlung gemäß Stufe (i) für eine Zeit von 0,1 bis 50 Stunden durchgeführt wird.

Die erfindungsgemäß eingesetzten Katalysatoren enthalten mindestens ein Übergangsmetall der 8. Nebengruppe des Periodensystems der Elemente, wobei auch Katalysatoren enthaltend zwei oder mehrere Übergangsmetalle oder Gemische aus zwei oder mehreren Katalysatoren enthaltend mindestens ein Übergangsmetall der 8. Nebengruppe des Periodensystems der Elemente eingesetzt werden können. Die eingesetzten Katalysatoren sind heterogen. Heterogene Katalysatoren können erfindungsgemäß suspendiert oder fest angeordnet sein.

Erfindungsgemäß geeignete heterogene Katalysatoren sind beispielsweise in Houben-Weyl, Methoden der Organischen Chemie, Band IV/1c, S. 16 bis 26, Thieme Verlag Stuttgart, 1980, beschrieben. Sie enthalten mindestens ein Übergangsmetall. Bevorzugte Übergangsmetalle sind Ni, Pd, Ru, Ir, Pt, Co und/oder Rh. Besonders bevorzugt sind Pd, Ru, Pt, ganz besonders bevorzugt sind Ru und Pd.

Die heterogenen Katalysatoren können in suspendierter Form oder bevorzugt fest angeordnet eingesetzt werden. Die Katalysatoren enthaltend mindestens ein Übergangsmetall der 8. Nebengruppe des Periodensystems der Elemente können das Übergangsmetall als Element oder in Form einer chemischen Verbindung, beispielsweise als Oxid enthalten. Mischungen von verschiedenen Übergangsmetallen können die Elemente oder deren Verbindungen als Mischungen oder als Legierungen enthalten. Dabei können auch nicht Übergangsmetall-haltige Elemente oder deren Verbindungen als Katalysatorkomponente verwendet werden, beispielsweise in den sogenannten Raney-Katalysatoren, wobei beispielsweise Al oder Aluminiumoxid, bevorzugt zusammen mit Ni, Cu oder Ru eingesetzt werden.

Erfindungsgemäß geeignet sind beispielsweise auch Ru auf Aluminium-, Silizium-, Titan-, Zirkon-, Barium-, Calziumoxid, Ru auf Aktivkohle, Pd auf Aluminium-, Silizium-, Titan-, Zirkon-, Barium-, Calziumoxid, Pd auf Aktivkohle, Pt auf Aluminium-, Silizium-, Titan-, Zirkon-, Barium-, Calziumoxid oder Pt auf Aktivkohle. Als Trägermaterialien können auch Mischungen oder Verbindungen verschiedener Materialen verwendet werden, beispielsweise Tonerden oder Zeolithe.

Die Katalysatoren enthaltend mindestens ein Übergangsmetall der Nebengruppe des Periodensystems der Elemente sind erfindungsgemäß auf einem Träger aufgebracht. Diese Träger sind beispielsweise metalloxidische, basische, neutrale oder saure Feststoffe, die erfindungsgemäß zur Erhöhung der Säurestärke beispielsweise mit Mineralsäuren wie Phosphorsäure oder Schwefelsäure behandelt worden sein können. Bevorzugt werden Oxide oder Mischoxide von B, Al, Si, Sn, Ti, Cr, Zr, Fe und Zn eingesetzt, die noch weitere Bestandteile enthalten können. Beispielsweise geeignet sind Zirkonoxid, Titanoxid, Aluminiumoxid, Siliziumoxid und Kombinationen daraus wie Alumosilikate, wie beispielsweise Zeolithe. Einsetzbar sind beispielsweise Schichtsilikate oder natürliche Tonerden.

Wird das erfindungsgemäße Verfahren mit heterogenen Katalysatoren diskontinuierlich durchgeführt, so wird in der Regel 0,1 bis 50 Gew.% Katalysator bezogen auf das cyclische Keton eingesetzt. Bevorzugt wird ein heterogener Katalysator in einer Menge von 0,5 bis 20 Gew.%, besonders bevorzugt von 1 bis 10 Gew.% eingesetzt.

Wird das Verfahren kontinuierlich mit einem heterogenen Katalysator durchgeführt, so wird vorzugsweise eine Katalysatorbelastung, d.h. die Belastung des heterogenen Katalysators, von 0,01 bis 10 kg Cyclododecanon/Liter Katalysator x h eingestellt. Insbesondere wird eine Katalysatorbelastung von 0,05 bis 2 kg Cyclododecanon/Liter Katalysator x h, besonders bevorzugt 0,1 bis 1 kg Cyclododecanon/Liter Katalysator x h eingestellt.

Gemäß einer bevorzugten Ausführungsform betrifft die vorliegende Erfindung daher ein wie zuvor beschriebenes Verfahren, wobei ein heterogener Katalysator mit einer Katalysatorbelastung von 0,01 bis 10 kg Cyclododecanon/Liter Katalysator x h eingesetzt wird.

Erfindungsgemäß ist es möglich, dass der heterogene, auf einem Träger aufgebrachte katalysator, der mindestens ein Übergangsmetall der 8. Nebengruppe des Periodensystems der Elemente enthält, im Schritt (ii) abgetrennt wird. Ebenso ist es jedoch im Rahmen der vorliegenden Erfindung möglich, dass der Katalysator nach Schritt (i) und vor Schritt (ii) abgetrennt wird. Mögliche Verfahren zur Abtrennung sind beispielsweise Destillation, Extraktion, Fällung oder Kristallisation.

Daher betrifft die vorliegende Erfindung gemäß einer weiteren Ausführungsform auch ein wie zuvor beschriebenes Verfahren, wobei nach Schritt (i) und vor Schritt (ii) der heterogene, auf einem Träger aufgebrachte Katalysator, der mindestens ein Übergangsmetall der 8. Nebengruppe des Periodensystems der Elemente enthält, zumindest teilweise abgetrennt wird.

Dabei enthält die Zusammensetzung (I') das Cyclododecanon üblicherweise in einer Menge von mehr als 80 Gew.%, vorzugsweise 85 bis 99 Gew.%, insbesondere 88 bis 99,9 Gew.%, besonders bevorzugt 90 bis 99,6 Gew.%, weiter bevorzugt 92 bis 99,0 Gew.-%. Die Zusammensetzung (I') enthält neben dem Cyclododecanon üblicherweise weitere Verbindungen, insbesondere organische Verbindungen, vorzugsweise solche mit sauerstoffhaltigen Gruppen, beispielsweise Alkohole, Aldehyde oder Epoxide, die vorzugsweise durch das erfindungsgemäße Reinigungsverfahren abgetrennt werden. Dabei können die organischen Verbindungen insbesondere dieselbe Anzahl an C-Atomen aufweisen wie das in der Zusammensetzung (I') enthaltene Cyclododecanon.

Die Nebenkomponenten sind in der Zusammensetzung (I') vor der Durchführung der erfindungsgemäßen Reinigung insbesondere zu weniger als 20 Gew.% enthalten, insbesondere weniger als 15 Gew.%, besonders bevorzugt weniger als 12 Gew.%. Beispielsweise sind die Nebenkomponenten in einer Menge von 0,001 bis 10 Gew.%, insbesondere von 0,1 bis 9 Gew.% enthalten, bevorzugt von 0,5 bis 5 Gew.%, insbesondere bevorzugt von 1 bis 4 Gew.%.

Daher betrifft die vorliegende Erfindung gemäß einer weiteren Ausführungsform auch ein wie zuvor beschriebenes Verfahren zur Reinigung einer Zusammensetzung (I) mindestens, wobei die Zusammensetzung (I') neben dem Cyclododecanon mindestens eine weitere sauerstoffhaltige organische Verbindung enthält.

Durch das erfindungsgemäße Verfahren wird Cyclododecanon in einer Reinheit von > 95 %, beispielsweise > 98 %, insbesondere > 99 %, ermittelt über gaschromatographische Methoden, erhalten. Vorzugsweise wird das Cyclododecanon in einer Reinheit von > 99,5 %, bevorzugt > 99,8 %, besonders bevorzugt > 99,9 % erhalten.

Erfindungsgemäß wird Cyclododecanon über eine Hydrierung von Cyclododecadienon gemäß Stufe (a3) erhalten, das wiederum durch Oxidation eines Cyclododecatriens, vorzugsweise mit Distickstoffmonoxid, gemäß Stufe (a2) erhalten wurde. Cyclododecatrien wird erfindungsgemäß durch Trimerisierung von Butadien gemäß Stufe (a1) erhalten. Erfindungsgemäß können zwischen den Stufen (a1), (a2) und (a3) weitere Behandlungen erfolgen, beispielsweise Reinigungsschritte.

Erfindungsgemäß erfolgt dabei zwischen den Stufen (a2) und (a3) kein Behandeln der in Stufe (a2) erhaltenen Zusammensetzung mit mindestens einer Base. Unter Behandeln wird in diesem Zusammenhang ein in Kontakt bringen der Zusammensetzung mit mindestens einer Base verstanden.

Stufe (a1) umfasst die Trimerisierung von Butadien. 1,5,9-Cyclododecatrien kann beispielsweise durch Trimerisierung von reinem 1,3-Butadien hergestellt werden, wie dies beispielsweise in T. Schiffer, G. Oenbrink, "Cyclododecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Cyclododecatrien kann durch Trimerisierung von 1,3-Butadien unter Verwendung eines Titan-Katalysators hergestellt werden.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders geeignet.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser Trimerisierung werden im Allgemeinen Gemische erhalten, die mindestens 95 Gew.-%, bevorzugt mindestens 96 Gew.-% und weiter bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die Umsetzung gemäß Stufe (a2) verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung gemäß Stufe (a2) einzusetzen.

Die Oxidation gemäß Stufe (a2) kann nach allen dem Fachmann bekannten geeigneten Verfahren erfolgen. Vorzugsweise wird die Oxidation gemäß Stufe (a2) im Rahmen des erfindungsgemäßen Verfahrens mittels Distickstoffmonoxid durchgeführt.

Gemäß Stufe (a2) wird Cyclododecatrien oxidiert, vorzugsweise durch Umsetzung mit Distickstoffmonoxid. Dabei kann für die Umsetzung des Cyclododecatriens mit Distickstoffmonoxid mindestens ein geeignetes Lösungsmittel oder Verdünnungsmittel eingesetzt werden. Als solche sind unter anderem Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung, noch eine C-C-Dreifachbindung, noch eine Aldehydgruppe aufweisen.

Im Allgemeinen ist bei der Umsetzung von Cyclododecatrien mit Distickstoffmonoxid der Zusatz eines Lösungsmittels oder Verdünnungsmittel nicht notwendig.

Die Temperaturen bei der Umsetzung von Cyclododecatrien mit Distickstoffmonoxid liegen bevorzugt im Bereich von 140 bis 350°C, weiter bevorzugt im Bereich von 180 bis 320°C und besonders bevorzugt im Bereich von 200 bis 300°C.

Es ist möglich, die Umsetzung von Cyclododecatrien mit Distickstoffmonoxid bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung von Cyclododecatrien mit Distickstoffmonoxid liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 300 bar und besonders bevorzugt im Bereich von 50 bis 200 bar.

Es ist möglich, die Umsetzung von Cyclododecatrien mit Distickstoffmonoxid bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Hinsichtlich der für die Umsetzung von Cyclododecatrien mit Distickstoffmonoxid einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Bevorzugt wird die Umsetzung von Cyclododecatrien mit Distickstoffmonoxid in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor bei der Umsetzung von Cyclododecatrien mit Distickstoffmonoxid liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit Cyclododecatrien zugeführt wird, liegt das Molverhältnis von Distickstoffmonoxid zu Cyclododecatrien im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Die Umsetzung von Cyclododecatrien mit Distickstoffmonoxid kann so durchgeführt werden, dass bei einer sehr hohen Selektivität bezüglich Cyclododecadienon ein Umsatz von Cyclododecatrien im Bereich von bis zu 50 %, bevorzugt im Bereich von 5 bis 30 % und insbesondere bevorzugt im Bereich von 10 bis 20 % erreicht wird. Dabei liegt die Selektivität, bezogen auf Cyclododecadienon, im Allgemeinen bei mindestens 90 %, bevorzugt bei mindestens 92,5 % und besonders bevorzugt bei mindestens 95 %.

Grundsätzlich kann jedes Cyclododecatrien oder jedes Gemisch aus zwei und mehr unterschiedlichen Cyclododecatrienen mit Distickstoffmonoxid umgesetzt werden. Unter anderem sind hierbei beispielsweise 1,5,9-Cyclododecatriene, beispielsweise cis,trans,trans-1,5,9-Cyclododecatrien oder cis,cis,trans-1,5,9-Cyclododecatrien oder all-trans-1,5,9-Cyclododecatrien, zu nennen.

Bevorzugt wird als Cyclododecatrien cis,trans,trans-1,5,9-Cyclododecatrien eingesetzt.

Im allgemeinen resultiert aus der Umsetzung von cis,trans,trans-1,5,9-Cyclododecatrien mit Distickstoffmonoxid ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon enthält. Ein beispielsweise typisches Isomerengemisch weist demgemäß die Isomeren in molaren Verhältnissen von in etwa 1 : 1 : 0,08 auf. Dies Isomerengemisch kann in der im erfindungsgemäßen Verfahren eingesetzten Zusammensetzung (I) enthalten sein.

Die Umsetzung von Cyclododecatrien mit Distickstoffmonoxid kann grundsätzlich in Anwesenheit eines Katalysators erfolgen, jedoch auch ohne Zusatz eines Katalysators.

Für die Hydrierung gemäß Stufe (a3) können sämtliche geeigneten Katalysatoren eingesetzt werden. Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß einsetzbaren Katalysatoren Pd, Pt, Ru oder Ni.

Geeignet sind beispielsweise homogene Katalysatoren enthaltend mindestens ein Element der 8., 9. oder 10. Nebengruppe. Weiter bevorzugt sind homogene Katalysatoren, die Ru, Rh, Ir und/oder Ni enthalten. Beispielsweise sind hierbei etwa RhCI(TTP)₃ oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind, deren diesbezügliche Offenbarung in den Kontext der vorliegenden Anmeldung vollumfänglich einbezogen wird. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH oder (TPP)₃(CO)RuCl₂.

Geeignet ist insbesondere mindestens ein heterogener Katalysator, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salzen oder Carbonaten ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 bis 700°C, insbesondere 100 bis 400°C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch-hydrierend wirkende Komponente beispielsweise durch Imprägnierung auf ein Trägermaterial aufgebracht worden ist.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600°C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817, EP 1 477 219 A1 oder EP 0 285 420 A1 auf den Träger aufgebracht werden. In den Katalysatoren gemäß den vorgenannten Schriften liegen die katalytisch aktiven Metalle als Legierungen vor, die durch thermische Behandlung und/oder Reduktion der z.B. durch Tränkung des Trägermaterials mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbare Katalysatoren dienen.

Ganz besonders bevorzugte Katalysatoren sind erfindungsgemäß solche, die Ni, Pt und/oder Pd enthalten und auf einem Träger aufgebracht sind. Ganz bevorzugte Träger sind oder enthalten Aktivkohle, Aluminiumoxid, Titandioxid und/oder Siliziumdioxid.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung gemäß Stufe (a3) mit mindestens einem Suspensionskatalysator durchgeführt, so wird bevorzugt in mindestens einem Rührreaktor oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihr Volumen und/oder ihren Querschnitt und/oder durch die Hydrierbedingungen in den Reaktoren unterscheiden.

Wird beispielsweise im Rahmen des erfindungsgemäßen Verfahrens die Hydrierung gemäß (a3) mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Wird als Katalysator bei der Hydrierung gemäß Stufe (a3) beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurück zu gewinnen.

Vor dem Einsatz in einem beliebigen Verfahren wie beispielsweise vor der Rückführung in das erfindungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärme kann bei dem erfindungsgemäß verwendeten Reaktor intern beispielsweise über Kühlschlangen und/oder extern beispielsweise über mindestens einen Wärmetauscher abgeführt werden. Wird beispielsweise bevorzugt mindestens ein Rohrreaktor zur Hydrierung eingesetzt, so wird bevorzugt die Reaktion über äußeren Umlauf gefahren, in dem die Wärmeabfuhr integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt genau zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung gemäß Stufe (a3) an mindestens einem suspendierten Katalysator durchgeführt, liegt die Verweilzeit im Allgemeinen im Bereich von 0,05 bis 50 h, beispielsweise im Bereich von 0,5 bis 50 h, bevorzugt im Bereich von 1 bis 30 h und besonders bevorzugt im Bereich von 1,5 bis 25 h, ganz besonders bevorzugt im Bereich von 1,5 bis 10 h. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Katalysatorbelastung (kg Feed/Liter Katalysator x h) im Allgemeinen im Bereich von 0,03 bis 20, bevorzugt im Bereich von 0,05 bis 5 und besonders bevorzugt im Bereich von 0,1 bis 2. Dabei ist es unerheblich, ob erfindungsgemäß ein Hauptreaktor und ein Nachreaktor oder zusätzlich weitere Reaktoren eingesetzt werden. Für sämtliche dieser Ausführungsformen liegt die Gesamtbelastung in den oben angegebenen Bereichen.

Allgemein liegt die Hydriertemperatur im Hauptreaktor im Bereich von 0 bis 350°C, bevorzugt im Bereich von 20 bis 300°C, weiter bevorzugt im Bereich von 50 bis 250°C und insbesondere bevorzugt im Bereich von 80 bis 220°C.

. Der Wasserstoffdruck liegt bei der erfindungsgemäßen Hydrierung im Hauptreaktor im Allgemeinen im Bereich von 1 bis 325 bar, bevorzugt im Bereich von 5 bis 300 bar, weiter bevorzugt im Bereich von 10 bis 250 bar und insbesondere bevorzugt im Bereich von 15 bis 150 bar.

Im Rahmen der erfindungsgemäßen Hydrierung gemäß Stufe (a3) kann mindestens ein geeignetes Lösungsmittel oder Verdünnungsmittel eingesetzt werden. Als solche sind grundsätzlich sämtliche Lösungs- und Verdünnungsmittel zu nennen, die unter den Hydrierbedingungen nicht hydriert oder anderweitig umgesetzt werden, z.B. Alkohole, Ether, Kohlenwasserstoffe, Wasser, Aromaten oder Ketone, insbesondere Toluol oder Cyclododecan.

Gemäß einer bevorzugten Ausführungsform des erfindungsgemäßen Verfahrens wird die Hydrierung gemäß Stufe (a3) ohne Zusatz eines Lösungs- oder Verdünnungsmittels durchgeführt.

Das erfindungsgemäße Verfahren zeichnet sich insbesondere dadurch aus, dass reine Produkte bei hohen Katalysatorstandzeiten und einer einfachen Verfahrensführung erhalten werden. Auch der apparative Aufbau für das erfindungsgemäße Verfahren ist einfach und kostengünstig.

Die Erfindung wird nachfolgend durch Beispiele näher erläutert.

### BEISPIELE

Die in den Beispielen angegebenen Gehalte sind gaschromatographisch bestimmte FI.-%. (GC-Methode)

### Beispiel 1:

Ein Cyclododecanon-Rohprodukt, hergestellt nach dem Verfahren wie in DE 103 44 595 A beschrieben, wobei bei der Hydrierstufe ein Cyclododecadienon eingesetzt wurde, das eine Reinheit von ca. 97,5 % aufweist, enthält 97 % Cyclododecanon sowie eine Vielzahl weiterer Komponenten, nämlich 0,2 % Cyclododecan, 0,5 % Cyclododecanol, 0,6 % Dodecanol, 0,3 % Dodecanal, 0,6 % Hydroxymethylcycloundecan, 0,1 % Formylcycloundecan sowie 0,2 % einer Vielzahl weitere Produkte, deren Einzelgehalte jeweils unter 0,05 % liegen. Dies Cyclododecanon-Rohprodukt wird an 100 ml eines Ru (5 %) auf Titandioxid bei 220 °C und einer Katalysatorbelastung von 0,5 kg Zulauf pro Liter Katalysator x h umgesetzt. Nach 24 Stunden Laufzeit wird der gesammelte Reaktionsaustrag anschließend in einer Kolonne bei 5 mbar fraktioniert destilliert. Man erhält Cyclododecanon in Reinheiten > 99,8 % mit ca. 97 % Destillationsausbeute.

### Vergleichsbeispiel 1:

Analog Beispiel 1 wird Cyclododecanon-Rohprodukt aufgearbeitet, allerdings ohne die Behandlung mit Katalysator. Man erhält Cyclododecanon in Reinheiten von 99,3 bis 99,5 % in einer Destillationsausbeute von ca. 60 %. Die restlichen Fraktionen enthalten Cyclododecanon mit Reinheiten von 60 bis 99,3 %.

## Patentansprüche

1. Verfahren zur Herstellung von Cyclododecanon, mindestens umfassend die Schritte
(a) Herstellung einer Zusammensetzung (I') mindestens enthaltend Cyclododecanon, mindestens umfassend die Stufen
(a-1) Trimerisierung von Butadien zu Cyclododecatrien
(a-2) Oxidation von Cyclododecatrien zu Cyclododecadienon
(a-3) Hydrierung von Cyclododecadienon zu Cyclododecanon
(b) Reinigung der Zusammensetzung (I'), mindestens umfassend die Stufen
(i) thermisches Behandeln der Zusammensetzung (I') mit mindestens einem heterogenen, auf einem Träger aufgebrachten Katalysator, der mindestens ein Übergangsmetall der 8. Nebengruppe des Periodensystems der Elemente enthält,
(ii) weiteres Aufreinigen durch ein Verfahren ausgewählt aus der Gruppe bestehend aus Destillation, Extraktion und Kristallisation.
wobei zwischen den Stufen (a-2) und (a-3) kein Behandeln der in Stufe (a-2) erhaltenen Zusammensetzung mit mindestens einer Base erfolgt.

2. Verfahren nach Anspruch 1, wobei die Behandlung gemäß Stufe (i) bei einer Temperatur von 60 bis 350 °C durchgeführt wird.

3. Verfahren nach einem der Ansprüche 1 oder 2, wobei die Behandlung gemäß Stufe (i) für eine Zeit von 0,1 bis 50 Stunden durchgeführt wird.

4. Verfahren nach einem der Ansprüche 1 bis 3, wobei die Zusammensetzung (I') neben dem mindestens einen cyclischen Keton mindestens eine weitere sauerstoffhaltige organische Verbindung enthält.

5. Verfahren nach einem der Ansprüche 1 bis 4, wobei das mindestens eine Übergangsmetall Ni, Pd, Ru, Ir, Pt, Co und/oder Rh ist.

6. Verfahren nach einem der Ansprüche 1 bis 5, wobei das mindestens eine Übergangsmetall Pd, Ru und/oder Pt ist.

7. Verfahren nach einem der Ansprüche 1 bis 6, wobei bei der destillativen Reinigung in (ii) bei Drücken zwischen 5 bis 500 mbar gearbeitet wird und wobei die Temperaturen (Sumpftemperatur) bei 100 bis 300 °C liegen.

8. Verfahren nach Anspruch 2, wobei die Behandlung gemäß Stufe (i) bei einer Temperatur von 130 bis 260 °C durchgeführt wird.

## Claims

1. A process for preparing cyclododecanone, which comprises at least the steps
(a) preparation of a composition (I') comprising at least cyclododecanone, which comprises at least the steps
(a-1) trimerization of butadiene to form cyclododecatriene,
(a-2) oxidation of cyclododecatriene to form cyclododecadienone,
(a-3) hydrogenation of cyclododecadienone to form cyclododecanone,
(b) purification of the composition (I'), which comprises at least the steps
(i) thermal treatment of the composition (I') with at least one heterogeneous catalyst which has been applied to a support and comprises at least one transition metal of transition group 8 of the Periodic Table of the Elements,
(ii) further purification by means of a process selected from the group consisting of distillation, extraction and crystallization,
where no treatment of the composition obtained in step (a-2) with at least one base is carried out between the steps (a-2) and (a-3).

2. The process according to claim 1, wherein the treatment in step (i) is carried out at a temperature of from 60 to 350°C.

3. The process according to claim 1 or 2, wherein the treatment in step (i) is carried out for a time of from 0.1 to 50 hours.

4. The process according to any of claims 1 to 3, wherein the composition (I') comprises at least one further oxygen-comprising organic compound in addition to the at least one cyclic ketone.

5. The process according to any of claims 1 to 4, wherein the at least one transition metal is Ni, Pd, Ru, Ir, Pt, Co and/or Rh.

6. The process according to any of claims 1 to 5, wherein the at least one transition metal is Pd, Ru and/or Pt.

7. The process according to any of claims 1 to 6, wherein the purification by distillation in (ii) is carried out at pressures in the range from 5 to 500 mbar and the temperatures (temperature at the bottom) are from 100 to 300°C.

8. The process according to claim 2, wherein the treatment in step (i) is carried out at a temperature of from 130 to 260°C.

## Revendications

1. Procédé de fabrication de cyclododécanone, comprenant au moins les étapes suivantes :
(a) la fabrication d'une composition (I') contenant au moins de la cyclododécanone, comprenant au moins les étapes suivantes :
(a-1) la trimérisation de butadiène en cyclododécatriène,
(a-2) l'oxydation de cyclododécatriène en cyclododécadiénone,
(a-3) l'hydrogénation de cyclododécadiénone en cyclododécanone,
(b) la purification de la composition (I'), comprenant au moins les étapes suivantes :
(i) le traitement thermique de la composition (I') avec au moins un catalyseur hétérogène appliqué sur un support, qui contient au moins un métal de transition du groupe de transition 8 du tableau périodique des éléments,
(ii) la purification supplémentaire par un procédé choisi dans le groupe constitué par la distillation, l'extraction et la cristallisation,
dans lequel aucun traitement de la composition obtenue à l'étape (a-2) avec au moins une base n'a lieu entre les étapes (a-2) et (a-3).

2. Procédé selon la revendication 1, dans lequel le traitement selon l'étape (i) est réalisé à une température de 60 à 350 °C.

3. Procédé selon l'une quelconque des revendications 1 ou 2, dans lequel le traitement selon l'étape (i) est réalisé pendant une durée de 0,1 à 50 heures.

4. Procédé selon l'une quelconque des revendications 1 à 3, dans lequel la composition (I') contient en plus de la ou des cétones cycliques au moins un autre composé organique contenant de l'oxygène.

5. Procédé selon l'une quelconque des revendications 1 à 4, dans lequel le ou les métaux de transition sont Ni, Pd, Ru, Ir, Pt, Co et/ou Rh.

6. Procédé selon l'une quelconque des revendications 1 à 5, dans lequel le ou les métaux de transition sont Pd, Ru et/ou Pt.

7. Procédé selon l'une quelconque des revendications 1 à 6, dans lequel la purification par distillation en (ii) est réalisée à des pressions comprises entre 5 et 500 mbar, les températures (température de fond) se situant dans la plage allant de 100 à 300 °C.

8. Procédé selon la revendication 2, dans lequel le traitement selon l'étape (i) est réalisé à une température de 130 à 260 °C.
